Europäisches Patentamt

European Patent Office

Office européen des brevets

(11) Publication number: **0 121 345**
**B1**

# EUROPEAN PATENT SPECIFICATION

(45) Date of publication of patent specification: **08.02.89**

(51) Int. Cl.⁴: **G 01 N 13/00**

(21) Application number: **84301406.9**

(22) Date of filing: **02.03.84**

(54) Dissolution testing machine.

(30) Priority: **04.03.83 GB 8306055**

(43) Date of publication of application:
**10.10.84 Bulletin 84/41**

(45) Publication of the grant of the patent:
**08.02.89 Bulletin 89/06**

(84) Designated Contracting States:
**AT BE CH DE FR IT LI LU NL SE**

(56) References cited:
**DE-B-2 409 222**
**DE-B-2 530 065**
**US-A-3 802 272**

(73) Proprietor: **Noormohammadi, Akbar**
**153 Earlsfield Road**
**London SW18 (GB)**
(73) Proprietor: **Beckett, Arnold Heyworth**
**16 Crestbrook Avenue**
**London N13 (GB)**
(73) Proprietor: **Geeves, Grahame Kenneth John**
**The Cobbles St Marys Road**
**Sunninghill Ascot Berkshire (GB)**
(73) Proprietor: **Dodd, Robert Edward**
**40 Firtree Avenue**
**Wallingford Oxon (GB)**

(72) Inventor: **Noormohammadi, Akbar**
**153 Earlsfield Road**
**London SW18 (GB)**
Inventor: **Beckett, Arnold Heyworth**
**16 Crestbrook Avenue**
**London N13 (GB)**
Inventor: **Geeves, Grahame Kenneth John**
**The Cobbles St Marys Road**
**Sunninghill Ascot Berkshire (GB)**
Inventor: **Dodd, Robert Edward**
**40 Firtree Avenue**
**Wallingford Oxon (GB)**

Courier Press, Leamington Spa, England.

**EP 0 121 345 B1**

(74) Representative: **Williams, John Francis et al**
**J.F. Williams & Co 34 Tavistock Street**
**London WC2E 7PB (GB)**

# EP 0 121 345 B1

## Description

This invention relates to dissolution testing machines, capable of measuring the dissolution rate of chemicals from tablets or pellets. Such machines are, in particular, used in the pharmaceutical industry in connection with measuring the controlled rate of release of drugs.

In the past, two main standard methods have been used to conduct experiments on the rate of release of chemicals. In the first, the tablets, capsules, pellets or the like (hereinafter called 'pellets'), are placed in the bottom of a standard container, and a paddle of standard shape is rotated about a vertical axis in a standard position in the container. However, the tendency is for the pellets to form a heap in the bottom of the container directly below the paddle, and the solution formed tends to maintain a gradient with the highest concentration at the bottom and the lowest at the top. Although standardised, as between tests such an apparatus does not provide a true indication of rate of dissolution of the pellets when taken internally by a patient.

In the second method, the pellets are placed inside a perforated basket of generally cylindrical shape, which basket is itself rotated about its central longitudinal axis in the liquid. However, the tendency then is to form a film of chemical in solution around the pellets, with a higher concentration of solution within the basket than outside it. An increase in the speed of rotation may increase the dissolution rate, but may also cause abrasion of the pellets by the basket mesh, which thus falsifies the results in an opposite direction.

A third unofficial method employs a rotating bottle, in effect mounted on an arm of a rotating wheel in a vertical plane, so that the bottle is tipped upside down once in every revolution. However, since the bottle must be liquid tight during this operation, it becomes very difficult to extract samples for testing at different time intervals, and the process is inconvenient and labour intensive.

DE—B—2 409 222 and DE—B—2 530 065 are not dissolution testers, but disintegration testers which have a different purpose. From these documents it can be seen that it is known to measure the disintegration rate of pellets by moving a holder up and down in a receptacle containing test liquid.

The invention aims to provide a machine which allows reproducible testing of the dissolution rate of pellets, permits sampling either manually or automatically, and which does not suffer, from the "retarding" effects common in known machines due to concentration gradients in the solvent due to inefficient flushing of the solvent over the pellets.

Accordingly, the invention proposes a dissolution testing machine comprising a container for a liquid, a holder for pellets open for passage of liquid at least at the bottom, means supporting the holder within the container and drive means enabling the holder to be reciprocated via its support means so that solvent flushes into and out of the holder, the bottom of the holder being soft and/or resilient so that pellets therein are not abraded thereby.

Such a machine preferably employs an elongated cylindrical container, similar to a test tube, and a cylindrical holder coaxial within the container and reciprocated along that axis. The holder may be a mesh basket, or a solid tube closed at its ends with mesh.

The invention also proposes a method of testing the dissolution rate of chemicals from pellets, wherein the chemicals are placed in a holder which is open at least at the bottom and has a bottom surface which is soft and/or resilient, and said holder is reciprocated up and down in a container of solvent in standard conditions, so that the solvent flushes into and out of the holder to dissolve the pellets but the latter are not abraded by contact with the bottom of the holder.

In a preferrzd method, said holder is moved successively from one to another of a plurality of containers, each container holding a solvent of the same or different characteristics.

In order that the invention shall be clearly understood, an exemplary embodiment of a machine will now be described with reference to the accompanying drawings, in which:

Figure 1 shows an overall schematic view of the machine according to the invention;

Figure 2 shows the support means and drive means, and

Figure 3 shows a container and a mesh basket holder for the machine.

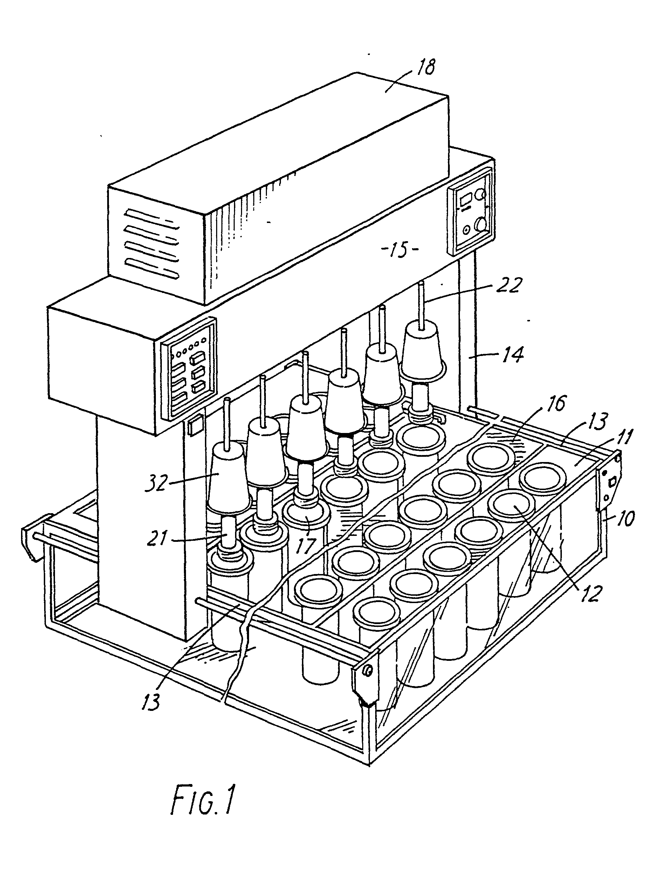

In Figure 1, a frame 10 supports a temperature controlled bath (not shown) which almost completely fills it. The top of the frame is covered by a matrix or framework 11 which defines a plurality of positions arranged in rows 16 and columns 17, each position 12 being designed to receive a glass container, approximately the shape and size of a large test tube. The bath is maintained at a constant temperature of approximately 37 degrees, thus maintaining the contents of the tubes at that temperature. A bridge structure 14 straddles the frame 10 and is movable by sliding along guide members on two upper sides 13 of the frame. Thus, the bridge 14 can be brought successively to positions above each of the rows of containers. The bridge 14 supports six mesh baskets (not shown) which are suspended below it, and also drive means 15 for controlling movement of the baskets.

A control unit 18 controls the operation of the machine.

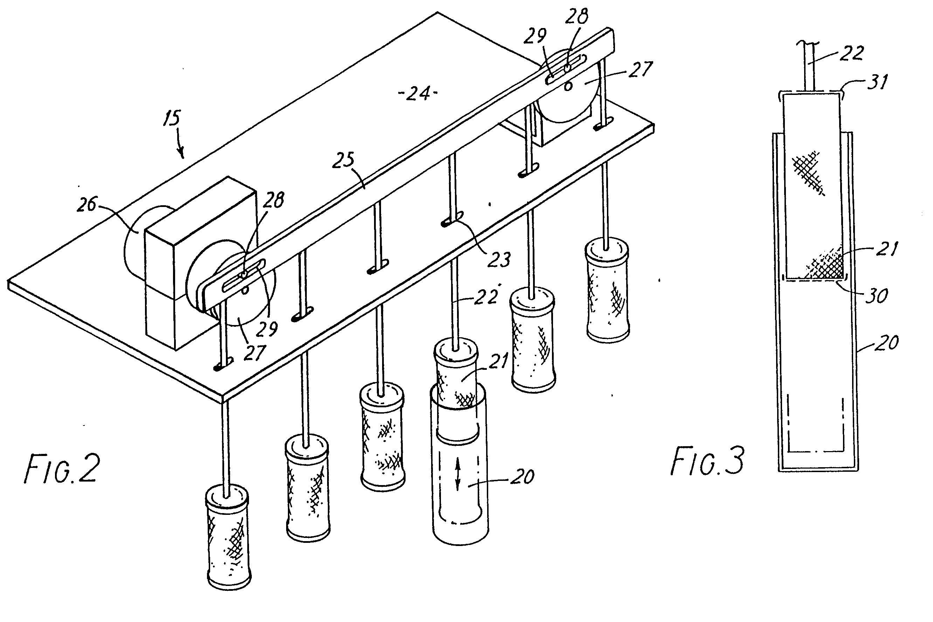

Figure 2 shows an enlarged and a more detailed view of the drive means and baskets supported on the bridge 14. A set of six glass containers 20 supported in the heated bath are arranged in the first row (only one container is shown). Directly above each container is suspended a small mesh basket 21, approximately 10 centimetres long and 2.5 cm in diameter. Each basket 21 is supported on a rod 22 which extends through a hole 23 in a support plate 24, and is fxied on a transverse bar 25. A motor 26 drives synchronously two discs 27 at a speed of between 5 and 50 r.p.m., preferably about 20 r.p.m. A pin 28 on each disc engages slots 29 in the beam 25, so that rotation of the discs 27 causes the whole structure supporting the series of baskets 21 to move gently up and down.

The nature of the container and basket can be seen from Figure 3. The container 20 is made of glass and has the shape of a right cylinder with an open top. The mesh basket 21 is made preferably of metal and is open at both top and bottom. The mesh may have any desired hole size. The bottom of the mesh basket is closed by a layer of gauze fabric 30 held by a small elastic band. The reason for this is that as the basket is reciprocated, the pellets will not be abraded by hitting the gauze. At the top, the basket is closed by a perforated plastic cap 31 which clips onto a bead on the top edge of the basket. The rod 22 is fixed directly to the cap 31. A splash cover 32 (Figure 1) may be provided. The materials used must be inert to both the solvent and the chemicals of the pellet, and must not absorb these substances.

In use, pellets to be dissolved are placed in the baskets, and the containers 20 are filled approximately three-quarters with a solvent, sufficient so that the pellets are not raised right out of the solvent. The up and down movement causes the solvent to be flushed in and out of the baskets in a controlled fashion, so as to produce dissolution of the pellets. The pellets are levitated with each downward stroke.

In an alternative, the hold may be a solid glasstube which cannot abrade the pellets, open top and bottom. These are then covered by soft mesh discs or fabric and held in position by a perforated plastic cap. Any suitable construction can be used which allows easy insertion of pellets, and easy liquid flow. The perforations of the cap and the mesh size of the disc or fabric may be chosen according to need. For example, very fine holes may be required for retard tablets which disintegrate before they dissolve. In every case, the bottom of the tube is to be formed by a soft and/or elastic material which will not abrade the pellets.

Since the controlled experiments normally to be carried out with the machine requires a series of tests of varying duration and with solvents of varying characteristics or concentrations, the machine is arranged so that the bridge 14, after performing one set of tests on the pellets in the first row of containers, is moved along the framework 10 until the baskets are aligned above the second row of containers. These containers may contain the same solvents at a different concentration, or a different solvent, and a further test can be performed for the same or a different time period. The dissolution rate in the first row of containers can be determined from the solution strength then remaining in those containers. Thus, the machine shown has the capacity to perform simultaneously six tests using the same or different pellets in different containers, and to repeat this operation six times in different solvents, or for different periods. Between each test, the baskets are raised to their highest position and the bridge 14 indexed sideways.

In a typical test, a single solvent is used in all cases, but with a different pH value in each row. The six successive tests would then be as follows:

| Row | 1 | 2 | 3 | 4 | 5 | 6 |
|------|------|------|------|------|------|------|
| pH | 1.5 | 4.5 | 6.9 | 6.9 | 7.2 | 7.5 |
| Time | 1 hr | 1 hr | 2 hrs | 2 hrs | 2 hrs | 4 hrs |

Such a test would approximate to the effect of gastric juices acting on a capsule swallowed by a person, and would be of use in determining the likely effects of swallowing a capsule containing a multitude of pellets of different solubilities intended to provide drug release throughout a period of 12 hours.

The measurement of the solution strengths left in each of the six containers forming one column and representing one set of test results can be performed in a number of ways. They can be tested manually as the sequence proceeds. Or the drive means and baskets can be replaced by a separate 'head' consisting of a set of six dip tubes which can sample and test all six containers of one row. By indexing the head sideways using the bridge 14, the dip tubes can be lowered into successive rows of containers and a set of readings produced for each column by each dip tube. Such a successive sampling can be carried out after completion of the actual dissolution tests.

Alternatively, the dip tubes could be mounted on the same head as the baskets, and arranged to sample the solutions one row behind the row currently receiving the pellets. In this case, the bridge 14 would need to index one step more than the numbers of rows of containers.

The control unit 18 may be manually controlled or micro-processor operated, so that all the indexing and timing can be pre-determined by hard or soft programming. This can also be applied to the subsequent sampling.

The invention can be applied to less complex machines than this, which is designed for batch sampling. A machine having one basket mounted on a head which is rotatable in the middle of a circular array of containers is also envisaged. Control may again be manual or automatic.

**Claims**

1. A dissolution testing machine comprising a container (20) for a liquid, a holder (21) for pellets open for passage of liquid at least at the bottom, means (14, 22) supporting the holder within the container and drive means (15) enabling the holder (21) to be reciprocated via its support means (22) so that solvent flushes into and out of the holder (21), the bottom (30) of the holder being soft and/or resilient so that pellets therein are not abraded thereby.

2. A machine as claimed in Claim 1, comprising a set of containers (20), in which said means (14, 15) can be moved so that the holder (21) is supported successively in each container (20) of the set.

3. A machine as claimed in Claim 2, wherein said set of containers (20) is located in a temperature controlled bath.

4. A machine as claimed in any preceding claim, wherein each said container (20) is tubular.

5. A machine as claimed in Claim 4 wherein each said holder (21) is a solid walled tube, closed top and bottom by an apertured member.

6. A machine as claimed in Claim 4 wherein each said holder (21) is a mesh-walled tube, closed top and bottom by an apertured member.

7. A machine as claimed in any of Claims 2 to 6 wherein said containers are arranged in a row (17) and said means (14, 15) can be moved linearly from one to the next.

8. A machine as claimed in Claim 7 wherein there are a plurality of sets of containers (20) and a corresponding plurality of holders (21), whereby a plurality of dissolution tests can be carried on simultaneously.

9. A machine as claimed in any of Claims 2 to 8 wherein a movable frame (14) above the containers carries the drive means (15), the support means (22) and the holders (21).

10. A machine as claimed in any of Claims 2 to 9 wherein a sampling tube can be arranged adjacent each said support means (22) for drawing off a sample of liquid in a container (21) either from the container in which the holder is supported or from an adjacent one of the set.

11. A machine as claimed in Claim 10 when appendant to Claim 9, wherein the movable frame (19) can be moved through a number of positions equal to one more than the number of containers (21) in the set.

12. A machine as claimed in any of Claims 2 to 6 wherein said set are arranged in a circle and said means (14, 15) can be moved in an arc from one to the next.

13. A method of testing the dissolution rate of chemicals from pellets, wherein the chemicals are placed in a holder (21), which is open at least at the bottom and has a bottom surface (30) which is soft and/or resilient; and said holder (21) is reciprocated up and down in a container (20) of solvent in standard conditions, so that the solvent flushes into and out of the holder (21) to dissolve the pellets but the latter are not abraded by contact with the bottom (30) of the holder (21).

14. A method as claimed in Claim 14 wherein said holder (21) is moved successively from one to another of a plurality of containers (20), each container (20) holding a solvent of the same or different characteristics.

## Patentansprüche

1. Auflösungs-Testmaschine, gekennzeichnet durch einen Behälter (20) für eine Flüssigkeit, eine Einsatz (21) für Kügelchen, der für den Durchlaß von Flüssigkeit zumindest am Boden offen ist, ein Halteelement (14, 21) das den Einsatz in dem Behälter trägt, und ein Antriebselement (15), durch welches der Einsatz (21) über sein Halteelement (22) derart hin und her bewegt wird, daß das Lösungsmittel in den Einsatz hinein- und aus diesem herausgespült wird, wobei der Boden (30) des Einsatzes (21) weich und/oder federnd ausgebildet ist, so daß die darin enthaltenen Kügelchen durch den Boden nicht abgerieben werden.

2. Maschine nach Anspruch 1, gekennzeichnet durch eine Gruppe von Behältern (20), in welcher das Element (14, 15) bewegbar ist, derart, daß der Einsatz (21) der Reihe nach in jedem Behälter (20) der Behaltergruppe gehalten wird.

3. Maschine nach Anspruch 2, dadurch gekennzeichnet, daß die Gruppe von Behältern (20) in einem temperaturgeregelten Bad angeordnet ist.

4. Maschine nach einem der Ansprüche 1 bis 3, dadurch gekennzeichnet, daß jeder Behälter (20) rohrförmig ist.

5. Maschine nach Anspruch 4, dadurch gekennzeichnet, daß jeder Einsatz (21) ein Rohr mit fester Wandung ist, das an Ober- und Unterseite mit einem Element verschlossen ist, das eine Öffnung aufweist.

6. Maschine nach Anspruch 4, dadurch gekennzeichnet, daß jeder Einsatz (21) ein Roh mit Gitterwandung ist, welches an Ober- und Unterseite durch ein Element verschlossen ist, das eine Öffnung aufweist.

7. Maschine nach einem der Ansprüche 2 bis 6, dadurch gekennzeichnet, daß die Behälter in einer Riehe (17) angeordnet sind und das Element (14, 15) linear von einem Behälter zum nächsten bewegt werden kann.

8. Maschine nach Anspruch 7, gekennzeichnet durch eine Vielzahl von Gruppen von Behältern (20) und eine entsprechende Vielzahl von Einsätzen (21), wodurch eine Vielzahl von Auflösungstests gleichzeitig durchgeführt werden kann.

9. Maschine nach einem der Ansprüche 2 bis 8, dadurch gekennzeichnet, daß ein beweglicher Rahmen (14) über den Behältern das Antriebselement (15), das Halteelement (22) und die Einsätze (21) trägt.

10. Maschine nach einem der Ansprüche 2 bis 9, dadurch gekennzeichnet, daß angrenzend an jedes Halteelement (22) ein Probenrohr angeordnet werden kann, nämlich zur Entnahme einer Probe der in dem Behälter (20) enthaltenen Flüssigkeit, und zwar aus demjenigen Behälter, in dem sich der Einsatz befindet, oder aus einem benachbarten Behälter der Gruppe.

11. Maschine nach Anspruch 10 bei Anbindung an Anspruch 9, dadurch gekennzeichnet, daß der bewegliche Rahmen (14) über eine Anzahl von Positionen hinweg bewegbar ist, die größer eins als die Anzahl der Behälter (20) in der Gruppe ist.

12. Maschine nach einem der Ansprüche 2 bis 6, dadurch gekennzeichnet, daß die Gruppe in einem Kreis angeordnet ist und das Element (14, 15) bogenförmigen von einem zum nächsten Behälter bewegt werden kann.

13. Verfahren zum Testen der Auflösungsrate von Chemikalien anhand von Kügelchen, dadurch gekennzeichnet, daß die Chemikalien in einem Einsatz (21) angeordnet werden, der zumindest am Boden offen ist und eine weiche und/oder federnde Bodenfläche (30) aufweist, und daß der Einsatz (21) in einem Lösungsmittelbehälter (20) bei Standardbedingungen hin und her bewegt wird, derart, daß das Lösungsmittel in den Einsatz hinein und aus dem Einsatz (21) herausgespült wird, so daß die Kügelchen aufgelöst, jedoch durch den Kontakt mit dem Boden (30) des Einsatzes (21) nicht abgerieben werden.

14. Verfahren nach Anspruch 14, dadurch gekennzeichnet, daß der Einsatz (21) der Reihe nach von einem zum anderen der Vielzahl von Behaltern (20) bewegt wird, wobei jeder der Behälter (20) ein Lösungsmittel mit den gleichen oder mit verschiedenen Eigenschaften enthält.

**Revendications**

1. Dispositif de mesure de dissolution comprenant un récipient (20) pour un liquide, un support (21) pour pellets ouvert au passage de liquid au moins un fond, des moyens (14, 22) pour supporter le support à l-intérieure du récipient et un moyen de commande (15) permettant d'appliquer au support (21) un mouvement de va-et-vient par l'intermédiaire de son moyen de support (22), de telle sorte que le solvant puisse balayer l'intérieur et l'extérieur du support (21), le fond (30) du support étant mou et/ou résilient de telle sorte que les pellets qui s'y trouvent ne s'usent pas ainsi par abrasion.

2. Dispositif suivant la revendicution 1, caractérisé en ce qu'il comprend au moins une série de récipients (20), dans lesquels les moyens (14, 15) peuvent être déplacés de telle sorte que le support (21) soit supporté successivement dans chaque récipient (20) de la série.

3. Dispositif suivant la revendication 2, caractérisé en ce que la série de récipients (20) est placée dans un bain à température contrôlée.

4. Dispositif suivant l'une quelconque des revendications précédentes, caractérisé en ce que chaque récipient (20) est tubulaire.

5. Dispositif suivant la revendication 4, caractérisé en ce que chaque support (21) est constitué par un tube à paroi pleines, fermé au sommet et au fond par un organe à ouvertures.

6. Dispositif suivant la revendication 4, caractérisé en ce que chaque support (21) est constitué par un tube à parois maillées, fermé au sommet et au fond par un organe à ouvertures.

7. Dispositif suivant l'une quelconque des revendications 2 à 6, caractérisé en ce que les récipients sont agencés en une rangée (17) et en ce que les moyens (14, 15) peuvent être déplacés linéairement de l'un à l'autre.

8. Dispositif suivant la revendication 7, caractérisé en ce qu'il y a une pluralité de séries de récipients (20) et une pluralité correspondante de supports (21), de sorte qu'une pluralité d'essais de dissolution peut être réalisée simultanément.

9. Dispositif suivant l'une quelconque des revendications 2 à 8, caractérisé en ce qu'une structure mobile (14) au-dessus du récipient support le moyen de commande (15), les moyens de support (22) et les supports (21).

10. Dispositif suivant l'une quelconque des revendications 2 à 9, caractérisé en ce qu'un tube d'échantillonage peut être agencé à proximité de chaque moyen de support (22) pour prélever un échantillon de liquide dans un récipient (21) soit à partir du récipient dans lequel le support est supporté soit à partir d'un récipient adjacent de la série.

11. Dispositif suivant la revendication 10, lorsque rattachée à la revendication 9, caractérisé en ce que la structure mobile (14) peut être déplacé sur un certain nombre de positions égale à un de plus que le nombre de récipients (21) de la série.

12. Dispositif suivant l'une quelconque des revendications 2 à 6, caractérisé en ce que la série est agencée en un cercle et en ce que les moyens (14, 15) peuvent être déplacés suivant un arc de l'un au suivant.

13. Procédé de mesure de la vitesse de dissolution de produits chimiques à partir de pellets, caractérisé en ce que les produits chimiques sont placés dans un support (21), qui est ouvert au moins au fond et qui a une surface de fond (30) qui est molle et/ou résiliente, et en ce que le support (21) est animé d'un mouvement vertical de va-et-vient dans un récipient (20) de solvant sous des conditions standards, de telle sorte que le solvant balie l'intérieur et l'extérieur du récipient (21) pour dissoudre les pellets mais que ces derniers ne soient pas usés par abrasion par contact avec le fond (30) du support (21).

14. Procédé suivant la revendication 13, caractérisé en ce que le support (21) est déplacé successivement d'un récipient à l'autre d'une pluralité de récipients (20) chaque récipient (20) contenant un solvant aux caractéristiques identiques ou différentes.

FIG.1

Fig.3

Fig.2